(19) Europäisches Patentamt
European Patent Office
Office européen des brevets

(11) **EP 2 908 126 B1**

(12) # EUROPÄISCHE PATENTSCHRIFT

(45) Veröffentlichungstag und Bekanntmachung des Hinweises auf die Patenterteilung:
**28.11.2018 Patentblatt 2018/48**

(51) Int Cl.:
**G01N 33/32** *(2006.01)*     **G01N 15/00** *(2006.01)*
**G01N 21/25** *(2006.01)*     *G01N 33/00* *(2006.01)*

(21) Anmeldenummer: **15400005.3**

(22) Anmeldetag: **05.02.2015**

(54) **Verfahren zur Bestimmung der Stabilität von Dispersionen**

Process for determining the stability of dispersions

Procédé pour la détermination de la stabilité de dispersion

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **15.02.2014 DE 102014002186**

(43) Veröffentlichungstag der Anmeldung:
**19.08.2015 Patentblatt 2015/34**

(73) Patentinhaber: **Orontec GmbH & Co. KG**
**44263 Dortmund (DE)**

(72) Erfinder:
• **Hustert, Hendrik**
**44225 Dortmund (DE)**

• **Wörheide, Ralph Jan**
**42369 Wuppertal (DE)**

(74) Vertreter: **Grundmann, Dirk et al**
**Rieder & Partner mbB**
**Patentanwälte - Rechtsanwalt**
**Postfach 11 04 51**
**42304 Wuppertal (DE)**

(56) Entgegenhaltungen:
**DE-A1- 2 525 701**     **US-A- 4 936 685**
**US-A1- 2013 141 727**     **US-B1- 6 292 264**

**Beschreibung**

Gebiet der Erfindung

Die Erfindung betrifft ein Verfahren und eine Vorrichtung zur Bestimmung der Stabilitat von Dispersionen, insbesondere von pigmentierten Dispersionen. Das Verfahren kann vorteilhaft eingesetzt werden zur Qualitätskontrolle bei der Herstellung und Verarbeitung von Dispersionen. Die Erfindung betrifft dariiber hinaus eine Verwendung der Vorrichtung.

Beschreibung des Standes der Technik

Es ist bekannt, dass in Dispersionen Wechselwirkungen zwischen den im Dispersionsmittel verteilten dispergierten Teilchen auftreten, deren Gleichgewicht eine homogene und stabile Dispersion garantiert. Ändern sich diese Wechselwirkungen, kann eine ursprünglich stabile Dispersion instabil werden, was sich z.B. in Flokkulation, Agglomeration, Aufschwimmen oder Absetzen äußern kann. Treten derartige Erscheinungen im Laufe des Herstellungs- oder Verarbeitungsprozesses von Dispersionen auf, so kann das negative Auswirkungen auf die Qualität des Endproduktes haben. Beispielsweise können sich technologische Eigenschaften von Dispersionsfarben, wie deren Deckkraft oder Applikationssicherheit, verschlechtern bzw. können die Dispersionsfarben gänzlich unbrauchbar werden. Bei der Herstellung von Dispersionen, insbesondere von pigmentierten Dispersionen, ist es daher erforderlich, dass sich die Stabilität einer Dispersion während des Herstellungsprozesses und nachfolgender Verarbeitungsprozesse nicht wesentlich verändert. In der Praxis werden jedoch während der Herstellung und auch der Weiterverarbeitung bzw. Lagerung der Dispersionen Stabilitätsschwankungen bzw. Stabilitätsabbau beobachtet. Es ist daher wünschenswert, bei der Herstellung und Verarbeitung von Dispersionen in den verschiedenen Stadien des Herstellungs- bzw. Verarbeitungsprozesses verlässliche Aussagen zur Stabilität einer Dispersion zu erlangen, um gegebenenfalls korrigierend in den jeweiligen Prozess eingreifen zu können.

Es sind verschiedene Verfahren bekannt, um farbmetrische Messungen an Nasslacken durchzuführen. Beispielsweise wird in US 6,292,264 B1 ein Verfahren beschrieben zum kontaktlosen Messen optischer Parameter einer Schicht aus einem flüssigen Überzugsmittel, welches auf ein umlaufendes Band appliziert wurde. Die optischen Parameter werden am Nasslack bestimmt, um feststellen zu können, ob das flüssige Überzugsmittel die geforderte Farbintensität aufweist. Weiterhin wird in US 2013/0141727 A1 ein Verfahren zur Messung des Flops einer Überzugsschicht in Abhängigkeit von der Zeit beschrieben. Aus den Flop-Messwerten wird dann der " degree of mottling" bestimmt. Im Verfahren geht es darum, den "degree of mottling" einer Überzugsschicht an Hand gemessener Flopwerte vorherzusagen, und zwar auf Basis eines vorab bestimmten funktionellen Zusammenhanges zwischen den Flopwerten einer Überzugsschicht im nassen Zustand und dem "degree of mottling" derselben Überzugsschicht im gehärteten Zustand.

DE-OS 2525701 offenbart ein Verfahren zur Bestimmung farbmetrischer Parameter von flüssigen Überzugsmitteln, wobei aus dem Überzugsmittel, z.B. auf einer rotierenden Scheibe, ein kontinuierlicher Film gebildet und dieser fortlaufend spektrophotometrisch gemessen wird. Des Weiteren offenbart US 4,936,685 ein Verfahren zur farbmetrischen Nasslackmessung, wobei die Farbparameter gemessen werden, während der Lack Scherkräften bzw. Turbulenzen ausgesetzt ist. Auf diese Art gemessene Farbwerte sollen gut korrelieren mit Farbwerten, die am getrockneten Überzug des gleichen Lackes gemessen werden.

Aussagen zur Stabilität bzw. zur Bestimmung der Stabilität einer Flüssigdispersion sind den vorstehend genannten Dokumenten nicht zu entnehmen.

Zusammenfassung der Erfindung

Aufgabe der vorliegenden Erfindung war es, ein Verfahren zur Verfügung zu stellen, welches es effektiv gestattet, Aussagen zur Stabilität von Dispersionen zu erlangen, beispielsweise in beliebigen Stadien ihres Herstellungs- und Verarbeitungsprozesses bzw. während Lagerung und Transport.

Erfindungsgemäß wurde die Aufgabe gelöst mit einem Verfahren zur Bestimmung der Stabilität von Dispersionen, wobei die Änderung optischer, zum Beispiel farbmetrischer, Parameter in Abhängigkeit von der Zeit als Maß für die Änderung der Stabilität einer Dispersion erfasst wird, sowie einer diesbezüglich eingerichteten Vorrichtung und die Verwendung einer Vorrichtung zur Durchführung des Verfahrens.

Die vorliegende Erfindung betrifft somit ein Verfahren zur Bestimmung der Stabilität von Dispersionen mittels optischer, zum Beispiel farbmetrischer, Messmethoden, umfassend die folgenden Schritte:

A) Bereitstellen einer zu untersuchenden Dispersion,

B) Bestimmen mindestens eines optischen, zum Beispiel farbmetrischen, Parameters der Dispersion im flüssigen Zustand in Abhängigkeit von der Zeit, und

C) Beurteilen der Stabilität der Dispersion anhand der bestimmten optischen, zum Beispiel farbmetrischen, Parameter,

wobei das Bestimmen des mindestens einen optischen Parameters der Flüssigdispersion im flüssigen Zustand in Schritt B) auf der Oberfläche eines sich in kontinuierlicher Bewegung befindlichen Trägers erfolgt.

Bevorzugt wird die Stabilität der Dispersion beurteilt auf Basis einer vorab definierten Toleranz und insbesondere bevorzugt auf Basis einer vorab definierten Toleranz bezüglich der Änderung der optischen, zum Beispiel farbmetrischen, Parameter in Abhängigkeit von der Zeit.

Überraschend wurde gefunden, dass die Korrelation zwischen Dispersionsstabilität pigmentierter Dispersionen und Änderung optischer, zum Beispiel farbmetrischer, Parameter in Abhängigkeit von der Zeit zu einer effektiven und verlässlichen Aussage über die Stabilität der jeweils zu untersuchenden Dispersion herangezogen werden kann. Es wird davon ausgegangen, dass die Änderung des Reflexionsverhaltens eines oder mehrerer Pigmente aus der lokalen Veränderung der Konzentration eines oder mehrerer dieser Pigmente in der Dispersion resultiert.

Detaillierte Beschreibung der Erfindung

Gemäß einer bevorzugten Ausführungsform betrifft die Erfindung ein Verfahren zur Bestimmung der Stabilität von Dispersionen mittels optischer, zum Beispiel farbmetrischer, Messmethoden, umfassend die folgenden Schritte:

A) Bereitstellen einer zu untersuchenden Dispersion,
B) Bestimmen mindestens eines optischen, zum Beispiel farbmetrischen, Parameters der Dispersion im flüssigen Zustand in Abhängigkeit von der Zeit, wobei die Dispersion auf die Oberfläche eines sich in kontinuierlicher Bewegung befindlichen Trägers aufgebracht und auf dem sich in kontinuierlicher Bewegung befindlichen Träger optisch, zum Beispiel farbmetrisch, gemessen wird, und
C) Beurteilen der Stabilität der Dispersion anhand der bestimmten optischen, zum Beispiel farbmetrischen, Parameter.

Gemäß einer weiteren bevorzugten Ausführungsform betrifft die Erfindung ein Verfahren zur Bestimmung der Stabilität von Dispersionen mittels optischer, zum Beispiel farbmetrischer, Messmethoden, umfassend die folgenden Schritte:

A) Bereitstellen einer zu untersuchenden Dispersion,
B) Bestimmen mindestens eines optischen, zum Beispiel farbmetrischen, Parameters der Dispersion im flüssigen Zustand in Abhängigkeit von der Zeit, wobei die Dispersion auf die Oberfläche eines sich in kontinuierlicher Bewegung befindlichen Trägers aufgebracht und auf dem sich in kontinuierlicher Bewegung befindlichen Träger optisch, zum Beispiel farbmetrisch, gemessen wird, und
C) Beurteilen der Stabilität der Dispersion anhand der bestimmten optischen, zum Beispiel farbmetrischen, Parameter,

wobei es sich bei dem in kontinuierlicher Bewegung befindlichen Träger um einen sich in kontinuierlicher Bewegung befindlichen scheibenförmigen Träger, bevorzugt um einen an einer rotierenden horizontalen Achse befestigten oder um einen um eine horizontale Achse rotierenden scheibenförmigen Träger handelt.

um einen an einer rotierenden horizontalen Achse befestigten oder um einen um eine horizontale Achse rotierenden scheibenförmigen Träger handelt.

Gemäß einer weiteren bevorzugten Ausführungsform betrifft die Erfindung ein Verfahren zur Bestimmung der Stabilität von Dispersionen mittels optischer, zum Beispiel farbmetrischer, Messmethoden, umfassend die folgenden Schritte:

A) Bereitstellen einer zu untersuchenden Dispersion,
B) Bestimmen mindestens eines optischen, zum Beispiel farbmetrischen, Parameters der Dispersion im flüssigen Zustand in Abhängigkeit von der Zeit, wobei die Dispersion auf die Oberfläche eines sich in kontinuierlicher Bewegung befindlichen Trägers aufgebracht und auf dem sich in kontinuierlicher Bewegung befindlichen Träger optisch, zum Beispiel farbmetrisch, gemessen wird, und
C) Beurteilen der Stabilität der Dispersion anhand der bestimmten optischen, zum Beispiel farbmetrischen, Parameter,

wobei zur Durchführung des Verfahrens eine Vorrichtung eingesetzt wird, umfassend:

a) ein Messgerät zur Messung optischer, zum Beispiel farbmetrischer, Parameter,
b) eine Küvette zur Aufnahme der Dispersion mit mindestens einer Einfüllöffnung und einer spaltförmigen Auslauföffnung, und
c) einen auf einer horizontalen Achse gelagerten, mit der Achse oder um die Achse drehbaren und in Bezug auf die Achse vertikal angeordneten scheibenförmigen Träger,

wobei die Küvette in Bezug auf den scheibenförmigen Träger so angeordnet ist, dass aus der spaltförmigen Auslauföffnung im Arbeitsmodus der Vorrichtung Dispersionsmaterial auf die Oberfläche des scheibenförmigen Trägers auslaufen kann.

Die vorstehend genannten bevorzugten Ausführungsformen können auch miteinander kombiniert werden.

Dabei kann die Küvette an den Seiten auf der Scheibe lagern und in der Mitte einen Spalt offen lassen.

Bevorzugt enthält die Vorrichtung auch eine Antriebsvorrichtung für den scheibenförmigen Träger oder für die horizontale Achse. Weiterhin enthält die Vorrichtung bevorzugt ein Gehäuse, welches die einzelnen Vorrichtungsbestandteile abdeckt. Bevorzugt handelt es sich um ein geschlossenes Gehäuse, das mit einer Klappe ausgestattet ist. Ebenfalls bevorzugt ist das Messgerät zur Messung optischer, zum Beispiel farbmetrischer, Parameter auf einer Positionierachse angeordnet, welche, beispielsweise elektrisch, angetrieben werden kann. Letzteres erlaubt es, das Messgerät an einem definierten Messpunkt entlang eines horizontalen Weges über dem Dispersionsfilm auf dem scheibenförmigen Träger zu positionieren. Die Vorrichtung erlaubt die berührungslose Messung des Dispersionsfilms.

Im Folgenden sollen die einzelnen Schritte des Verfahrens der vorliegenden Erfindung näher erläutert werden.

In Schritt A des erfindungsgemäßen Verfahrens wird eine Dispersion bereitgestellt. Eine Dispersion ist ein System aus mehreren Phasen, von denen eine kontinuierlich (Dispersionsmittel) und mindestens eine weitere fein verteilt ist (dispergierte Phase). Im Sinne der Erfindung handelt es sich um sogenannte Flüssigdispersionen, d.h. um Dispersionen bei denen das Dispersionsmittel eine Flüssigkeit ist und die disperse Phase eine Flüssigkeit und/oder ein Feststoff ist. Dispersionen bei denen eine flüssige Phase in einer flüssigen Phase dispergiert ist, nennt man auch Emulsionen, Dispersionen bei denen eine feste Phase in einer flüssigen Phase dispergiert ist, nennt man auch Suspensionen. Zur Vereinfachung werden die in der vorliegenden Erfindung eingesetzten Flüssigdispersionen hier und im Folgenden Dispersionen genannt.

Bei den Dispersionen kann es sich um pigmentierte oder nicht pigmentierte Dispersionen handeln. Pigmentierte Dispersionen sind bevorzugt mit farbgebenden und/oder effektgebenden Pigmenten pigmentiert. Es kann sich um wasserbasierende Dispersionen, um Dispersionen auf Basis von organischen Lösemitteln oder um Dispersionen handeln, die frei von Wasser und organischen Lösemitteln sind. Beispiele für pigmentierte Dispersionen sind Dispersionsfarben, Pigmentpasten und pigmentierte Lacke. Beispiele für nicht pigmentierte Dispersionen sind flüssige Bindemittelzubereitungen oder nicht pigmentierte Lacke. Es können jedoch auch beliebige andere pigmentierte oder nicht pigmentierte Dispersionen im Verfahren eingesetzt werden. Nicht pigmentierte Dispersionen können zum Beispiel durch Zugabe von farbgebenden und/oder effektgebenden Pigmenten und/oder Farbstoffen für das Verfahren aufbereitet werden.

Die zu untersuchende Dispersion wird beispielsweise an beliebiger gewünschter Stelle ihres Herstellungs- oder Verarbeitungsprozesses entnommen oder anderweitig bereitgestellt, beispielsweise kann es sich auch um eine von einem Hersteller bezogene Dispersion handeln. Das erfindungsgemäße Verfahren kann für Dispersionen beliebiger Pigmentierung angewendet werden. Die Dispersionen können farbgebende und/oder effektgebende Pigmente enthalten. Beispiele für farbgebende Pigmente sind Absorptionspigmente (z. B. Irgazin® Orange L 2990 HD, Irgazin® Red L 3660HD, Heliogen Blau L 7080 und BAYFERROX® 3920).

Beispiele für effektgebende Pigmente sind Reflexionspigmente (z.B. STAPA® HYDROLAN 2156, STAPA® HYDROLAN S 2100, Aquasilber LPW/980 oder Aquamet NPW/2900) oder Interferenzpigmente (z.B. Iriodin® 9225 Rutile Blue Pearl SW, Colorstream® T20-03 WNT, Tropic Sunrise und SunGEM® 288-7222).

Die Pigmente können einzeln oder in Kombination miteinander vorhanden sein. Die Dispersion kann auch Farbstoffe enthalten.

Schritt B

In Schritt B des erfindungsgemäßen Verfahrens werden die optischen, zum Beispiel farbmetrischen, Parameter der Dispersion im flüssigen Zustand in Abhängigkeit von der Zeit bestimmt. Handelt es sich bei der zu untersuchenden Dispersion um eine nicht pigmentierte, d.h. um eine nicht mit farbgebenden und/oder effektgebenden Pigmenten pigmentierte Dispersion, muss diese Dispersion zunächst durch Zugabe von farbgebenden und/oder effektgebenden Pigmenten und/oder Farbstoffen aufbereitet werden. Das kann beispielsweise durch Zugabe einer speziellen pigmentierten Prüfpaste (z.B. Pehafix Pigmentlösung rot, Peter-Lacke GmbH) oder einer Farbstofflösung (z.B. Pehazell Farbstofflösung rot, Peter-Lacke GmbH) zur Dispersion erfolgen.

Um die optischen, zum Beispiel farbmetrischen, Parameter der Dispersion im flüssigen Zustand zu bestimmen, wird die Dispersion auf einen geeigneten Träger appliziert und im flüssigen Zustand mittels eines geeigneten Messgerätes, beispielsweise mittels eines Spektralphotometers, gemessen. Zur Messung wird die Dispersion bevorzugt auf die Oberfläche eines sich in kontinuierlicher Bewegung befindlichen Trägers, insbesondere auf die Oberfläche eines sich in kontinuierlicher Bewegung befindlichen scheibenförmigen Trägers aufgebracht und auf dem sich in kontinuierlicher Bewegung befindlichen Träger gemessen. Es sind verschiedene Methoden zur optischen, zum Beispiel farbmetrischen, Charakterisierung von flüssigen Medien, z.B. von Flüssiglacken, auf bewegten Trägern bekannt. Beispielsweise kann die Applikation auf ein sich in kontinuierlicher Bewegung befindliches Band, auf einen sich in kontinuierlicher Bewegung befindlichen zylindrischen Körper, z.B. eine rotierende Walze, oder auf einen sich in kontinuierlicher Bewegung befindlichen scheibenförmigen Träger, z.B. eine rotierende Messscheibe, erfolgen. Ein Verfahren zur farbmetrischen Charakterisierung von flüssigen Medien mit Hilfe eines sich in kontinuierlicher Bewegung befindlichen Bandes ist z.B. beschrieben in DE 30 29 257 A1. Hier wird das flüssige Medium mittels einer Auftragswalze und verschiedener Verteilerwalzen aufgebracht. Ein Verfahren zur farbmetrischen Charakterisierung mit Hilfe einer rotierenden Walze ist z.B. beschrieben in DE 199 60 919 A1. Hier wird das flüssige Medium mittels einer Küvette mit spaltförmiger Öffnung zur Regelung der Schichtdicke des Films auf einen kontinuierlich rotierenden walzenförmigen Träger aufgebracht. Die DE 25 25 701 A1 beschreibt ein Verfahren zur farbmetrischen Charakterisierung von flüssigen Medien mit Hilfe eines mit einer Unterlage wandernden Lackfilms, wie zum Beispiel einer um eine horizontale Achse rotierenden Messscheibe.

Gemäß einer bevorzugten Ausführungsform wird die Dispersion auf einen sich in kontinuierlicher Bewegung befindlichen scheibenförmigen Träger aufgebracht, besonders bevorzugt auf einen um eine horizontale Achse rotierenden scheibenförmigen Träger oder auf einen aneiner rotierenden horizontalen Achse befestigten scheibenförmigen Träger.

Der scheibenförmige Träger kann eine Messscheibe, beispielsweise aus gehärtetem Stahl, sein.

Generell kann die Applikation der Dispersion auf die Oberfläche eines sich in kontinuierlicher Bewegung befindlichen Trägers mit geeigneten Mitteln, beispielsweise mittels eines Behälters, wie z.B. einer Küvette, erfolgen, wobei dieser bzw. diese entsprechende Einfüll- und Auslassöffnungen aufweisen. Vorzugsweise wird in der vorstehend genannten bevorzugten Ausführungsform die Dispersion mittels einer Küvette mit spaltförmiger Öffnung zur Regelung der Schichtdicke des applizierten Films auf den sich in kontinuierlicher Bewegung befindlichen scheibenförmigen Träger aufgebracht. Die Schichtdicke kann dabei beispielsweise 100 $\mu$m bis 1500 $\mu$m betragen.

Nach Applikation werden die gewünschten optischen, zum Beispiel farbmetrischen, Parameter des applizierten Dispersionsfilms, z.B. spektralphotometrisch, gemessen bzw. erfasst. Die Messung erfolgt dabei an dem Dispersionsfilm, der sich auf dem in kontinuierlicher Bewegung befindlichen Träger befindet, wobei der Träger vorzugsweise ein um eine horizontale Achse rotierender scheibenförmiger Träger, z.B. eine Messscheibe, ist. Geeignete zu bestimmende optische Parameter sind zum Beispiel farbmetrische Parameter, der Brechungsindex, die Verteilung von Bildpixeln und die Glitzerdichte. Geeignete farbmetrische Parameter sind die Reflexionswerte oder Remissionswerte. Ebenso können als farbmetrische Parameter auch aus den Reflexionswerten oder Remissionswerten abgeleitete Parameter verwendet werden.

Die optischen, zum Beispiel farbmetrischen, Parameter, z.B. die Reflexionswerte oder daraus abgeleitete Parameter, werden mittels üblicher spektralphotometrischer Standardmessgeräte erfasst. Derartige Messgeräte sind dem Fachmann hinlänglich bekannt und im Handel erhältlich.

Die optischen, zum Beispiel farbmetrischen, Parameter werden erfindungsgemäß in Abhängigkeit von der Zeit erfasst. Das beinhaltet insbesondere eine Messung der optischen, zum Beispiel farbmetrischen, Parameter der applizierten Dispersion in Abhängigkeit von der Zeit, wobei sich die Dispersion während der Dauer der Gesamtmesszeit auf dem sich in kontinuierlicher Bewegung befindlichen Träger befindet. Das heißt, die Messungen in Abhängigkeit von der Zeit erfolgen, während sich der Träger in kontinuierlicher Bewegung befindet. Gemäß einer Ausführungsform kann das erfolgen, indem die optischen, zum Beispiel farbmetrische, Parameter der auf den sich in kontinuierlicher Bewegung befindlichen Träger applizierten Dispersion in definierten Zeitabständen gemessen bzw. bestimmt werden. Die Messungen können beispielsweise bis zu einer Gesamtdauer von 30 Sekunden bis 20 Minuten erfolgen. Die Messungen können innerhalb der gewählten Gesamtmessdauer in Abständen von beispielsweise 10 Sekunden bis 3 Minuten erfolgen. Zur Messung wird das Messgerät, z.B. ein Spektralphotometer, so vor den sich in kontinuierlicher Bewegung befindlichen Träger, z.B. einer um eine horizontale Achse rotierenden Scheibe, positioniert, dass eine berührungslose Messung an der gewählten Messposition erfolgen kann. Sofern mehrere Betrachtungswinkel verwendet werden, wird die Messung für jeden Betrachtungswinkel durchgeführt.

Als farbmetrische Parameter können zum Beispiel die Remissionswerte, Reflexionswerte, die Farbkoordinaten (CIEL*a*b*-Werte), der Farbabstand E* bei gewähltem Betrachtungs- und Beleuchtungswinkel, das Chroma C*, der Bunttonwinkel h* bzw. die Änderung dieser Parameter über die Zeit erfasst werden. Die Änderung eines farbmetrischen Parameters beinhaltet dabei die Differenz des Wertes des farbmetrischen Parameters zwischen dem Nullwert (Wert des farbmetrischen Parameters zum Zeitpunkt Null) und dem Wert zum Zeitpunkt x der Messung. Die gemessenen bzw. ermittelten farbmetrischen Parameter können in einer Datenbank gespeichert und durch eine Software ausgewertet werden. Analoges gilt auch generell für optische Parameter.

Vorteilhafterweise kann das Messgerät, z.B. das Spektralphotometer, so ausgestattet bzw. programmiert sein, dass für jeden Messpunkt direkt die Differenz des Wertes des optischen, zum Beispiel farbmetrischen, Parameters zwischen dem Nullwert und dem Wert zum Zeitpunkt x der Messung ausgegeben wird. Gut geeignet ist zum Beispiel die Änderung des Farbabstandes Delta E*.

Remissions- und Reflexionsmessungen sowie die Berechnung bzw. Ableitung von Farbwerten aus den gemessenen Werten sind dem Fachmann bekannt. Dies kann nach solchen Normen erfolgen, wie sie für die Trockenlackmessung verwendet werden. Beispiele für solche Normen sind:
DIN 5033-1 (1979-03-00) Farbmessung; Grundbegriffe der Farbmetrik; DIN 5033-2 (1992-05-00) Farbmessung; Normvalenz-Systeme; DIN 5033-3 (1992-07-00) Farbmessung; Farbmaßzahlen; DIN 5033-4 (1992-07-00) Farbmessung; Spektralverfahren; DIN 5033-5 (1981-01-00) Farbmessung; Gleichheitsverfahren; ISO 7724-1 Lacke und Anstrichstoffe; Farbmessung; Teil 1: Grundlagen; ISO 7724-2 (1984-10-00) Lacke und Anstrichstoffe; Farbmessung; Teil 2: Bestimmung von Farbmaßzahlen; ISO 7724-3 (1984-10-00) Lacke und Anstrichstoffe; Farbmessung; Teil 3: Berechnung von Farbabständen.

Schritt C

In Schritt C des erfindungsgemäßen Verfahrens wird die Stabilität der Dispersion anhand der gemessenen bzw. bestimmten optischen, zum Beispiel farbmetrischen, Parameter beurteilt, vorzugsweise auf Basis der Differenz des optischen, zum Beispiel farbmetrischen, Parameters zwischen dem Nullwert (Wert des optischen, zum Beispiel farbmetrischen, Parameters zum Zeitpunkt Null) und dem Wert zum Zeitpunkt x der Messung.

Die Auswertung erfolgt durch Ermittlung einer Regression, vorzugsweise einer linearen Regressionsgeraden, die den gewählten optischen, zum Beispiel farbmetrischen, Parameter (z.B. den Farbabstand E*) bzw. dessen Änderung in Abhängigkeit von der Zeit darstellt.

Die Bewertung der erhaltenen Daten kann durch Vergleich der Parameter der Regressionsfunktion, z. B. bei einer Regressionsgeraden durch die Steigung $m_1$ und den Achsenabschnitt der Ordinate $m_0$ (d.h. dem Schnittpunkt der Regressionsgeraden mit der Ordinate) sowie der Parameter für die Güte der Kurvenanpassung, beispielsweise das Bestimmtheitsmaß $r^2$, erfolgen. Dabei gilt für die Parameter $m_0$ (Achsenabschnitt der Ordinate) und $m_1$ (Steigung der Regressionsgeraden), dass sie möglichst nahe an Null liegen sollen, d. h., kleinere Werte deuten auf eine stabile Dispersion, größere Werte auf eine weniger stabile Dispersion hin. Für die Parameter der Güte der Kurvenanpassung gilt, dass sich eine schlechtere Güte der Anpassung durch kleinere Werte äußert und auf eine instabile Dispersion hindeutet. Für eine stabile Dispersion sollte das Bestimmtheitsmaß $r^2$ mindestens 0,97 (entsprechend 97%) betragen. Dabei können die Daten sowohl auf der Basis der grafischen Darstellung als auch der numerischen Faktoren aus der Berechnung der Regressionsgeraden und der Güte der Kurvenanpassung verglichen werden.
d(L*, a*, b*, C, h, E*)$_{(Betrachtungswinkel, Beleuchtungswinkel)}$ = $m_0$ + ($m_1$ x Zeit), d.h. $m_0$ + ($m_1$ multipliziert mit der Zeit (in Stunden, Minuten, Sekunden)).

Dabei soll $m_0$ vorzugsweise Null sein. Je kleiner $m_0$ ist, desto stabiler ist die Dispersion. Bevorzugt ist $m_0$ maximal $\pm$ 0,05.

Vorzugsweise soll auch $m_1$ Null sein. Je kleiner $m_1$ ist, desto stabiler ist die Dispersion. Bevorzugt ist $m_1$ zum Beispiel maximal 0,1 $min^{-1}$ für den Gesamtfarbabstand dE*. Zur Bestimmung der Güte der Kurvenanpassung kann beispielsweise folgende Beziehung verwendet werden:

$$r^2 = 1 - \frac{\sum Fehler^2}{\sum Gesamt^2} = 1 - \frac{\sum (y_i - \hat{y}_i)^2}{\sum (y_i - \bar{y}_i)^2}$$

$r^2$ = Bestimmtheitsmaß

$$\sum Fehler^2 = \sum (y_i - \hat{y}_i)^2$$

$$\sum Gesamt^2 = \sum (y_i - \bar{y}_i)^2$$

$y_i$ = i - ter beobachteter Antwortwert
$\hat{y}_i$ = i - ter angepaßter Antwortwert
$\bar{y}_i$ = Mittelwert Antwort

Die oben genannte Beziehung kann auch als gewichtete Funktion verwendet werden:

$$r^2 = 1 - \frac{\sum (y_i - \hat{y}_i)^2}{\sum (y_i - \bar{y}_i)^2} * \left( \frac{(n-1)}{(n-p-1)} \right)$$

n = Anzahl der Datenpunkte
p = Anzahl der Terme des Regressionsmodells

$$r^2 = 1 - \frac{\sum (y_i - \hat{y}_i)^2}{\sum (y_i - \bar{y}_i)^2} * f(z)$$

$f(z)$ = Beliebige Form einer Wichtungsfunktion

Bevorzugt erfolgt die Beurteilung der Stabilität der Dispersion auf Basis einer vorab definierten Toleranz, beispielsweise einer vorab definierten Toleranz bezüglich der Änderung der optischen, zum Beispiel farbmetrischen, Parameter in Abhängigkeit von der Zeit oder der Steigung der Regressionsgeraden. Die noch akzeptable Toleranz kann individuell festgelegt und an die spezifischen Umstände und Erfordernisse angepasst werden.

Wünschenswert ist generell eine lineare Abhängigkeit der Änderung der optischen, zum Beispiel farbmetrischen, Parameter (z.B. der Änderung des Farbabstandes Delta E*) sowie eine Regressionsgerade mit Steigung nahe Null. Eine lineare Abhängigkeit bzw. eine geringe Steigung der Regressionsgeraden weisen auf eine Dispersion mit ausreichender bzw. akzeptabler Stabilität.

Übersteigt die Änderung der optischen, zum Beispiel farbmetrischen, Parameter, beispielsweise die Änderung des Farbabstandes mit der Zeit, eine bestimmte vorab definierte Toleranz, ergibt dies einen Hinweis auf eine instabile Dispersion oder zumindest eine Dispersion mangelnder Stabilität. Anhand solcher Ergebnisse können dann geeignete Maßnahmen ergriffen werden, um die Stabilität der Dispersion zu erhöhen oder wieder herzustellen. Das kann beispielsweise durch Zugabe, Entfernen und/oder Austausch bestimmter Pigmente, Additive und/oder weiterer Inhaltsstoffe der Dispersion erfolgen. Natürlich kann sich auch die Unbrauchbarkeit einer Dispersion in Bezug auf Stabilität herausstellen.

Die Erfindung betrifft, wie vorstehend bereits beschrieben, auch eine Vorrichtung zur Durchführung des erfindungsgemäßen Verfahrens. Eine bevorzugte Vorrichtung zur Durchführung des erfindungsgemäßen Verfahrens ist beschrieben in DE 20 2013 009 407 U1. Derartige Vorrichtungen sind auch kommerziell erhältlich, z.B. als Kompaktvorrichtung unter dem Namen Q-Chain® LCM.

Das erfindungsgemäße Verfahren und die Vorrichtung können eingesetzt werden bei der Herstellung und Verarbeitung von pigmentierten und nicht pigmentierten Dispersionen, zum Beispiel in der Lack- und Farbenindustrie. Mit Hilfe des Verfahrens können wertvolle Informationen über die Stabilität von Dispersionen gewonnen werden. Es kann beispielsweise sowohl in der Entwicklung als auch bei der Qualitätskontrolle von Pigmentzubereitungen Anwendung finden. Dazu werden zum Beispiel Pasten mit einem weißen Prüflack in einem definierten Verhältnis abgemischt und die Änderung der optischen, zum Beispiel farbmetrischen, Parameter in Abhängigkeit von der Zeit gemessen.

Das erfindungsgemäße Verfahren soll an Hand des folgenden Beispiels dargestellt werden.

Beispiel

Als zu untersuchende Dispersion wurde eine Mischung aus einem weißen Prüflack und einer Farbpaste, mit Pigment Red 254 pigmentiert, verwendet. Zur Applikation und Messung wurde ein Kompaktgerät Q-Chain® LCM eingesetzt. Die Dispersion wurde in die Küvette der Messvorrichtung gefüllt und auf die rotierende Scheibe (Umdrehung: 1 Hz) appliziert. Es ergab sich eine Schichtdicke von ca. 900 $\mu$m. Dann wurde das Messgerät mit der Messöffnung an einen Messpunkt in der unteren Hälfte der Scheibe herangeführt und die Änderung des Farbabstandes Delta E* (45/0-Geometrie) in Abständen von circa einer Minute innerhalb einer Gesamtmessdauer von etwa 14 Minuten gemessen. Die ermittelten Messwerte sind in Tabelle 1 aufgeführt.

Tabelle 1:

| Zeit [hh:mm:ss] | dE*$_{45/0}$ |
|---|---|
| 00:00:00 | 0,00 |
| 00:01:11 | 0,08 |
| 00:02:14 | 0,20 |
| 00:03:16 | 0,27 |
| 00:04:18 | 0,30 |
| 00:05:25 | 0,37 |

(fortgesetzt)

| Zeit [hh:mm:ss] | dE*$_{45/0}$ |
|---|---|
| 00:06:32 | 0,41 |
| 00:07:43 | 0,48 |
| 00:08:50 | 0,57 |
| 00:09:58 | 0,66 |
| 00:11:05 | 0,68 |
| 00:12:11 | 0,76 |
| 00:13:32 | 0,84 |
| dE=Delta E; 45/0 = Messgeometrie 45°/0° | |

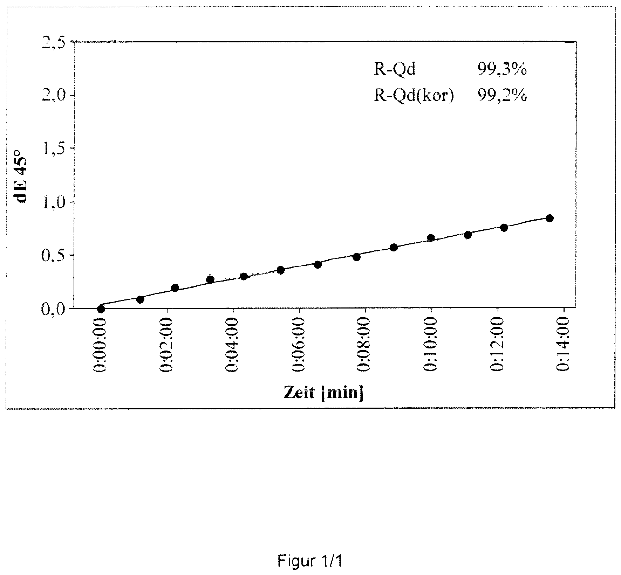

Die Auswertung der Messwerte erfolgte anhand einer Regressionsgeraden. In Figur 1 ist die Anpassungsgerade dargestellt. Aus der Darstellung ist ersichtlich, dass die Steigung $m_{1, dE}$ = 0,07 min$^{-1}$ und der Ordinatenabschnitt $m_{0, dE}$ = 0,03 betragen. Das bedeutet, dass es sich bei der untersuchten Dispersion um eine stabile Dispersion handelt.

**Patentansprüche**

1. Verfahren zur Bestimmung der Stabilität von Flüssigdispersionen mittels optischer Messmethoden, umfassend die folgenden Schritte:

   A) Bereitstellen einer zu untersuchenden Flüssigdispersion,
   B) Bestimmen mindestens eines optischen Parameters der Flüssigdispersion im flüssigen Zustand in Abhängigkeit von der Zeit, und
   C) Beurteilen der Stabilität der Flüssigdispersion anhand des bestimmten mindestens einen optischen Parameters,

   wobei das Bestimmen des mindestens einen optischen Parameters der Flüssigdispersion im flüssigen Zustand in Schritt B) auf der Oberfläche eines sich in kontinuierlicher Bewegung befindlichen Trägers erfolgt.

2. Verfahren nach Anspruch 1, wobei es sich um eine pigmentierte Flüssigdispersion handelt.

3. Verfahren nach Anspruch 1 oder 2, wobei es sich bei dem mindestens einen optischen Parameter um einen farbmetrischen Parameter handelt.

4. Verfahren nach Anspruch 3, wobei es sich bei dem farbmetrischen Parameter um einen Reflexionswert, einen Remissionswert oder einen aus einem Reflexionswert oder Remissionswert abgeleiteten farbmetrischen Parameter handelt.

5. Verfahren nach Anspruch 4, wobei es sich bei dem aus einem Reflexionswert oder Remissionswert abgeleiteten farbmetrischen Parameter um die Farbkoordinaten (CIEL*a*b*-Werte), den Farbabstand E*, das Chroma C* und/oder den Bunttonwinkel h* handelt.

6. Verfahren nach einem der Ansprüche 1 bis 5, wobei es sich bei dem in kontinuierlicher Bewegung befindlichen Träger um einen scheibenförmigen Träger handelt.

7. Verfahren nach Anspruch 6, wobei es sich um einen um eine horizontale Achse rotierenden scheibenförmigen Träger oder um einen an einer rotierenden horizontalen Achse befestigten scheibenförmigen Träger handelt.

8. Verfahren nach einem der Ansprüche 1 bis 7, wobei die Flüssigdispersion mittels einer Küvette mit spaltförmiger Öffnung zur Regelung der Schichtdicke des applizierten Films auf den sich in kontinuierlicher Bewegung befindlichen Träger aufgebracht wird.

9. Verfahren nach einem der Ansprüche 1 bis 8, wobei zur Durchführung des Verfahrens eine Vorrichtung eingesetzt wird, umfassend:

   a) ein Messgerät zur Messung optischer Parameter,
   b) eine Küvette zur Aufnahme der Flüssigdispersion mit mindestens einer Einfüllöffnung und einer spaltförmigen Auslauföffnung, und
   c) einen auf einer horizontalen Achse gelagerten, mit der Achse oder um die Achse drehbaren und in Bezug auf die Achse vertikal angeordneten scheibenförmigen Träger,

   wobei die Küvette in Bezug auf den scheibenförmigen Träger so angeordnet ist, dass aus der spaltförmigen Auslauföffnung im Arbeitsmodus der Vorrichtung Dispersionsmaterial auf die Oberfläche des scheibenförmigen Trägers auslaufen kann.

10. Vorrichtung, umfassend:

   a) ein Messgerät zur Messung optischer Parameter;
   b) eine Küvette zur Aufnahme der Flüssigdispersion mit mindestens einer Einfüllöffnung und einer spaltförmigen Auslauföffnung, und
   c) einen auf einer horizontalen Achse gelagerten, mit der Achse oder um die Achse drehbaren und in Bezug auf die Achse vertikal angeordneten scheibenförmigen Träger,

wobei die Küvette in Bezug auf den scheibenförmigen Träger so angeordnet ist, dass aus der spaltförmigen Auslauföffnung im Arbeitsmodus der Vorrichtung Dispersionsmaterial auf die Oberfläche des scheibenförmigen Trägers auslaufen kann,
wo es einen Dispersionsfilm bildet,
**dadurch gekennzeichnet,**
**dass** das Messgerät elektrisch angetrieben an einen definierten Messpunkt entlang eines horizontalen Weges über den Dispersionsfilm auf dem scheibenförmigen Träger positionierbar ist, um in definierten Zeitabständen auf dem sich in kontinuierlicher Bewegung befindlichen Dispersionsfilm die optischen Parameter zu messen, wobei das Messgerät derart programmiert ist, dass für jeden Messpunkt die Differenz des optischen Parameters zu einem Nullwert ausgegeben wird.

11. Verwendung einer Vorrichtung umfassend:

   a) ein Messgerät zur Messung optischer Parameter;
   b) eine Küvette zur Aufnahme der Flüssigdispersion mit mindestens einer Einfüllöffnung und einer spaltförmigen Auslauföffnung, und
   c) einen auf einer horizontalen Achse gelagerten, mit der Achse oder um die Achse drehbaren und in Bezug auf die Achse vertikal angeordneten scheibenförmigen Träger,

wobei die Küvette in Bezug auf den scheibenförmigen Träger so angeordnet ist, dass aus der spaltförmigen Auslauföffnung im Arbeitsmodus der Vorrichtung Dispersionsmaterial auf die Oberfläche des scheibenförmigen Trägers auslaufen kann,
zur Durchführung eines Verfahren zur Bestimmung der Stabilität von Flüssigdispersionen mittels optischer Messmethoden, umfassend die folgenden Schritte:

   A) Bereitstellen einer zu untersuchenden Flüssigdispersion,
   B) Bestimmen mindestens eines optischen Parameters der Flüssigdispersion im flüssigen Zustand in Abhängigkeit von der Zeit, und
   C) Beurteilen der Stabilität der Flüssigdispersion anhand des bestimmten mindestens einen optischen Parameters,

wobei das Bestimmen des mindestens einen optischen Parameters der Flüssigdispersion im flüssigen Zustand in Schritt B) auf der Oberfläche eines sich in kontinuierlicher Bewegung befindlichen Trägers erfolgt.

**Claims**

1. Method for determining the stability of liquid dispersions by means of optical measurement methods, comprising the following steps:

   A) providing a liquid dispersion to be examined,
   B) determining at least one optical parameter of the liquid dispersion in the liquid state as a function of time, and
   C) assessing the stability of the liquid dispersion on the basis of the at least one determined optical parameter,

   wherein the at least one optical parameter of the liquid dispersion in the liquid state is determined in step B) on the surface of a continuously moving substrate.

2. Method according to claim 1, wherein said dispersion is a pigmented liquid dispersion.

3. Method according to either claim 1 or claim 2, wherein the at least one optical parameter is a colourimetric parameter.

4. Method according to claim 3, wherein the colourimetric parameter is a reflectance value, a remission value or a colourimetric parameter derived from a reflectance value or a remission value.

5. Method according to claim 4, wherein the colourimetric parameter derived from a reflectance value or remission value is the colour coordinates (CIEL*a*b* values), the colour distance E*, the chroma C* and/or the hue angle h*.

6. Method according to any of claims 1 to 5, wherein the continuously moving substrate is a disc-shaped substrate.

7. Method according to claim 6, wherein said substrate is a disc-shaped substrate that rotates about a horizontal shaft or is a disc-shaped substrate that is fastened to a rotating horizontal shaft.

8. Method according to any of claims 1 to 7, wherein the liquid dispersion is applied to the continuously moving substrate by means of a cuvette having a gap-like opening for controlling the layer thickness of the applied film.

9. Method according to any of claims 1 to 8, wherein a device is used to carry out the method, which device comprises:

   a) a measuring apparatus for measuring optical parameters,
   b) a cuvette for receiving the liquid dispersion having at least one inlet opening and one gap-like outlet opening, and
   c) a disc-shaped substrate that is mounted on a horizontal shaft, is rotatable by means of the shaft or about the shaft and is arranged vertically with respect to the shaft,

   wherein the cuvette is arranged with respect to the disc-shaped substrate such that, in the operating mode of the device, dispersion material can leak out of the gap-like outlet opening onto the surface of the disc-shaped substrate.

10. Device comprising:

   a) a measuring apparatus for measuring optical parameters;
   b) a cuvette for receiving the liquid dispersion having at least one inlet opening and one gap-like outlet opening, and
   c) a disc-shaped substrate that is mounted on a horizontal shaft, is rotatable by means of the shaft or about the shaft and is arranged vertically with respect to the shaft,

   the cuvette being arranged with respect to the disc-shaped substrate such that, in the operating mode of the device, dispersion material can leak out of the gap-like outlet opening onto the surface of the disc-shaped substrate,
   on which substrate said material forms a dispersion film,
   **characterised in that** the measuring apparatus can be positioned on the disc-shaped substrate in an electrically driven manner at a defined measuring point along a horizontal path over the dispersion film in order to measure, in defined time intervals, the optical parameters on the continuously moving dispersion film, the measuring apparatus being programmed such that the difference between the optical parameter and a zero value is output for each measuring point.

11. Use of a device comprising:

   a) a measuring apparatus for measuring optical parameters;
   b) a cuvette for receiving the liquid dispersion having at least one inlet opening and one gap-like outlet opening, and
   c) a disc-shaped substrate that is mounted on a horizontal shaft, is rotatable by means of the shaft or about the shaft and is arranged vertically with respect to the shaft,

   wherein the cuvette is arranged with respect to the disc-shaped substrate such that, in the operating mode of the device, dispersion material can leak out of the gap-like outlet opening onto the surface of the disc-shaped substrate,

for carrying out a method for determining the stability of liquid dispersions by means of optical measurement methods, comprising the following steps:

A) providing a liquid dispersion to be examined,
B) determining at least one optical parameter of the liquid dispersion in the liquid state as a function of time, and
C) assessing the stability of the liquid dispersion on the basis of the at least one determined optical parameter,

wherein the at least one optical parameter of the liquid dispersion in the liquid state is determined in step B) on the surface of a continuously moving substrate.

**Revendications**

1. Procédé de détermination de la stabilité de dispersions liquides au moyen de procédés de mesure optiques, comprenant les étapes suivantes :

A) fourniture d'une dispersion liquide à analyser,
B) détermination d'au moins un paramètre optique de la dispersion liquide à l'état liquide en fonction du temps, et
C) évaluation de la stabilité de la dispersion liquide sur la base de l'au moins un paramètre optique déterminé,

dans lequel la détermination de l'au moins un paramètre optique de la dispersion liquide à l'état liquide dans l'étape B) a lieu à la surface d'un support continuellement en mouvement.

2. Procédé selon la revendication 1, dans lequel il s'agit d'une dispersion liquide pigmentée.

3. Procédé selon la revendication 1 ou 2, dans lequel l' au moins un paramètre optique est un paramètre colorimétrique.

4. Procédé selon la revendication 3, dans lequel le paramètre colorimétrique est une valeur de réflexion, une valeur de réflectance ou un paramètre colorimétrique dérivé d'une valeur de réflexion ou d'une valeur de réflectance.

5. Procédé selon la revendication 4, dans lequel le paramètre colorimétrique dérivé d'une valeur de réflexion ou de réflectance consiste en les coordonnées de couleur (valeurs CIEL*a*b*), l'écart colorimétrique E*, la chrominance C* et/ou l'angle de teinte h*.

6. Procédé selon l'une quelconque des revendications 1 à 5, dans lequel le support continuellement en mouvement est un support en forme de disque.

7. Procédé selon la revendication 6, dans lequel il s'agit d'un support en forme de disque tournant autour d'un axe horizontal ou d'un support en forme de disque fixé à un axe horizontal rotatif.

8. Procédé selon l'une des revendications 1 à 7, dans lequel la dispersion liquide est appliquée au moyen d'une cuvette avec une ouverture en forme de fente pour régler l'épaisseur de couche du film appliqué sur le support continuellement en mouvement.

9. Procédé selon l'une des revendications 1 à 8, dans lequel est utilisé pour la mise en oeuvre du procédé un dispositif comprenant :

a) un appareil de mesure pour mesurer un paramètre optique,
b) une cuvette pour recevoir la dispersion liquide avec au moins une ouverture de remplissage et une ouverture de décharge en forme de fente, et
c) un support en forme de disque monté sur un axe horizontal, pouvant tourner avec l'axe ou autour de l'axe et agencé verticalement par rapport à l'axe ;

dans lequel la cuvette est agencée par rapport au support en forme de disque de manière que, dans le mode de fonctionnement du dispositif, du matériau de dispersion puisse s'écouler sur la surface du support en forme de disque depuis l'ouverture de décharge en forme de fente.

10. Appareil, comprenant :

a) un appareil de mesure pour mesurer un paramètre optique ;
b) une cuvette pour recevoir la dispersion liquide avec au moins une ouverture de remplissage et une ouverture de décharge en forme de fente, et
c) un support en forme de disque monté sur un axe horizontal, pouvant tourner avec l'axe ou autour de l'axe et agencé verticalement par rapport à l'axe ;

dans lequel la cuvette est disposée par rapport au support en forme de disque de manière que la cuvette est agencée par rapport au support en forme de disque de manière que, dans le mode de fonctionnement du dispositif, du matériau de dispersion puisse s'écouler sur la surface du support en forme de disque depuis l'ouverture de décharge en forme de fente,
où il forme un film de dispersion,
**caractérisé en ce que**

l'appareil de mesure peut être positionné par entraînement électrique au niveau d'un point de mesure défini le long d'un trajet horizontal au-dessus du film de dispersion sur le support en forme de disque pour mesurer à des intervalles de temps définis les paramètres optiques sur le film de dispersion continuellement en mouvement, dans lequel l'appareil de mesure est programmé de manière à fournir, pour chaque point de mesure, la différence du paramètre optique par rapport à une valeur nulle.

11. Utilisation d'un dispositif comprenant :

a) un appareil de mesure pour mesurer un paramètre optique ;
b) une cuvette pour recevoir la dispersion liquide avec au moins une ouverture de remplissage et une ouverture de décharge en forme de fente, et
c) un support en forme de disque monté sur un axe horizontal, pouvant tourner avec l'axe ou autour de l'axe et agencé verticalement par rapport à l'axe ;

dans laquelle la cuvette est disposée par rapport au support en forme de disque de manière que la cuvette est agencée par rapport au support en forme de disque de manière que, dans le mode de fonctionnement du dispositif, du matériau de dispersion puisse s'écouler sur la surface du support en forme de disque depuis l'ouverture de décharge en forme de fente,
pour la mise en oeuvre d'un procédé de détermination de la stabilité de dispersions liquides au moyen de procédés de mesure optiques, comprenant les étapes suivantes :

A) fourniture d'une dispersion liquide à analyser,
B) détermination d'au moins un paramètre optique de la dispersion liquide à l'état liquide en fonction du temps, et
C) évaluation de la stabilité de la dispersion liquide sur la base de l'au moins un paramètre optique déterminé,

dans lequel la détermination de l'au moins un paramètre optique de la dispersion liquide à l'état liquide dans l'étape B) a lieu à la surface d'un support continuellement en mouvement.

Figur 1/1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6292264 B1
- US 20130141727 A1
- DE OS2525701 A
- US 4936685 A
- DE 3029257 A1
- DE 19960919 A1
- DE 2525701 A1
- DE 202013009407 U1